# EUROPÄISCHE PATENTSCHRIFT

(11) **EP 2 433 651 B1**
(45) Veröffentlichungstag und Bekanntmachung des Hinweises auf die Patenterteilung: **20.03.2013**
(21) Anmeldenummer: 10175300.2
(22) Anmeldetag: 03.09.2010
(51) Int. Cl.: A61K 47/10, G01N 33/15

(54) **Bestimmungsmethode für Aldehyde und Ketone in Glycerin**
Method for detecting aldehydes and ketones in glycerine
Méthode de détermination pour des aldéhydes et de cétones dans la glycérine

(43) Veröffentlichungstag der Anmeldung: 28.03.2012
(73) Patentinhaber: Aug. Hedinger GmbH & Co. KG, 70327 Stuttgart (DE)
(72) Erfinder: Milek, Frank, Dr., 70619 Stuttgart (DE); Rouven, Josl, Dr., 73760 Ostfildern (DE)
(74) Vertreter: Köster, Hajo

(56) Entgegenhaltungen:
- EP-B1- 1 242 121
- US-A1- 2008 033 191
- CANCHO B ET AL: "Determination of aldehydes in drinking water using pentafluorobenzylhydroxylamine derivatization and solid-phase microextraction", JOURNAL OF CHROMATOGRAPHY, ELSEVIER SCIENCE PUBLISHERS B.V, NL, Bd. 943, Nr. 1, 11. Januar 2002 (2002-01-11), Seiten 1-13, XP004329784, ISSN: 0021-9673, DOI: 10.1016/S0021-9673(01)01437-6
- GONCALVES L M ET AL: "Analysis of aldehydes in beer by gas-diffusion microextraction: Characterization by high-performance liquid chromatography-diode-array detection-atmospheric pressure chemical ionization-mass spectrometry", JOURNAL OF CHROMATOGRAPHY, ELSEVIER SCIENCE PUBLISHERS B.V, NL, Bd. 1217, Nr. 24, 11. Juni 2010 (2010-06-11), Seiten 3717-3722, XP027054168, ISSN: 0021-9673 [gefunden am 2010-04-09]

## Beschreibung

Die Erfindung betrifft ein Verfahren zur quantitativen Bestimmung von Verunreinigungen in Form von Aldehyden und Ketonen in für die Arzneimittelherstellung dienendem Glycerin, bei dem das die Verunreinigungen enthaltende Glycerin in einer Probelösung mit einem Derivatisierungsreagenz umgesetzt und die Menge an derivatisierten Verunreinigungen bestimmt werden.

Arzneimittel müssen bekanntlich strenge Qualitätsanforderungen erfüllen, um an den Verbraucher beziehungsweise den Patienten abgegeben werden zu können. Die in den Arzneimitteln enthaltenden Stoffe und ihre Darreichungsformen müssen den anerkannten pharmazeutischen Regeln entsprechen, welche u. A. in Arzneibüchern definiert sind.

Glycerin gehört zu denjenigen Ausgangsstoffen, die häufig bei der Arzneimittelherstellung eingesetzt werden. Für diese Chemikalie Glycerin existieren Monographien in verschieden Arzneibüchern, beispielsweise im europäischen Arzneibuch (Ph. Eur.), dem Amerikanischen Arzneibuch (USP) und dem Japanischen Arzneibuch (JP). Glycerin muss den in diesen Arzneibüchern niedergelegten Spezifikationen entsprechen.

Glycerin ist allerdings oxidationsempfindlich. Sowohl bei der Herstellung als auch bei Lagerung von Glycerin können Aldehyde und Ketone entstehen. Das Auftreten derartiger Verunreinigungen in Glycerin kann bei dem Einsatz von Glycerin zur Herstellung von Arzneimitteln zu Qualitätsproblemen führen.

Die in den oben erwähnten Monographien beschriebenen Verfahren zur Bestimmung von Verunreinigungen in Form von Aldehyden und Ketonen sind jedoch unzulänglich. Lediglich das Europäische Arzneibuch enthält eine explizite Spezifikation für Aldehyde. Gemäß dieser Spezifikation wird der Aldehydgehalt durch Derivatisierung mit Pararosaniliniumchlorid und durch anschließende UV/VIS-Messung bestimmt, wobei Formaldehyd als Referenz dient. Gemäß dieser Spezifikation darf der Aldehydgehalt maximal 10 ppm betragen.

In der USP werden Aldehyde nur durch die gaschromatographische Untersuchung auf "Related Compounds" erfasst; die Spezifikationsgrenze liegt dabei bei maximal 0,1 %.

Glycerin kann Aldehyde und Ketone in Gehalten bis über 60 ppm enthalten und dennoch die Anforderungen bei der Prüfung gemäß dem Europäischen Arzneibuch erfüllen. Dies liegt daran, dass die Methode des Europäischen Arzneibuchs unpräzise ist und lediglich Formaldehyd erfasst, jedoch die anderen Aldehyde und Ketone nur unzureichend. Für den Arzneimittelhersteller besteht daher das Risiko, dass der von ihm bezogene Ausgangsstoff Glycerin zwar die Prüfung gemäß Europäischem Arzneibuch besteht, jedoch der wahre Gehalt an Aldehyden und Ketonen nicht lediglich 10 ppm beträgt, sondern wesentlich darüber liegt. Von diesem hohen Gehalt an Aldehyden und Ketonen erlangt der Arzneimittelhersteller somit keine Kenntnis. Aufgrund der Reaktivität von Aldehyden und Ketonen kann dies jedoch negative Auswirkungen auf die Qualität des fertigen Arzneimittels haben.

Diese Problematik wird auch in der EP 1 242 121 B1 diskutiert. Dort befindet sich eine zusammenfassende Darstellung der verschiedenen Problemstellungen und auch ein Testverfahren zur Bestimmung des Gehalts von reaktiven Aldehyden in Glycerinproben.

Bei diesem bekannten Verfahren handelt es sich um einen kolorimetischen Essay, bei dem Glycerinaldehyd als Standard eingesetzt wird. Bei diesem bekannten Verfahren wird 3-Methyl-2-benzothiazolinon-hydrazon-hydrochlorid (MBTH) eingesetzt. Nach erfolgter Umsetzung wird die Absorption der Reaktionslösung bei 624 nm spektrophotometrisch gemessen.

In dieser Druckschrift wird auch dieser MBTH-Test mit anderen bekannten Tests verglichen. Nachteilig an diesen bekannten Verfahren ist der Umstand, dass damit nur einige der Verunreinigungen von Glycerin quantitativ detektiert werden können

Aufgabe der vorliegenden Erfindung ist es daher, ein Verfahren bereit zu stellen, mit dem möglichst viele und insbesondere alle Verunreinigungen in Form von Aldehyden und Ketonen in Glycerin besser quantitativ bestimmt werden können.

Gelöst wird diese Aufgabe durch ein Verfahren gemäß der Lehre der Ansprüche.

Bei dem erfindungsgemäßen Verfahren zur quantitativen Bestimmung von Aldehyden und Ketonen wird als Derivatisierungsreagenz PFBHA eingesetzt. Es handelt sich dabei um O-(2,3,4,5,6-pentafluorobenzyl)-hydroxylamin hydrochlorid.

Erfindungsgemäß wird somit eine Probelösung, welche das zu untersuchende Glycerin enthält, mit dem genannten Derivatisierungsreaganz, das vorzugsweise in einer gepufferten wässrigen Derivatisierungslösung vorliegt, versetzt.

Ferner wird ein Lösungsvermittler in Form eines polaren organischen insbesondere wasserlöslichen Lösungsmittels hinzugegeben. Dabei kann es sich beispielsweise um Alkohole mit 1 bis 5 C-Atomen, insbesondere um Monoalkohole mit 1 bis 5 C-Atomen, sowie um Acetonitril handeln, wobei Acetonitril bevorzugt ist.

Bei dem erfindungsgemäßen Verfahren werden das Glycerin und die darin enthaltenen Verunreinigungen in einer Probelösung mit dem Derivatisierungsreagenz umgesetzt.

Bei dieser Umsetzung bzw. Reaktion werden die oben genannten Verunreinigungen in die entsprechenden Derivate überführt. Die nach der Umsetzung erhaltene Probelösung und somit die derivatisierten Verunreinigungen werden dann flüssigchromatographisch aufgetrennt.

Die Derivatisierung bzw. Umsetzung erfolgt vorzugsweise in einem thermostatisierten Raum. Bei diesem Raum handelt es sich vorzugsweise um einen Autosampler. Die Derivatisierung kann beispielsweise bei einer Temperatur von 0 - 76°C, vorzugsweise bei 0 - 60°C, weiterhin vorzugsweise bei 20-35°C und insbesondere bevorzugt bei ca. 25°C durchgeführt werden.

Die angegebenen Temperaturbereiche 0-76 °C umfassen alle dazwischen liegenden, insbesondere ganzzahligen Temperaturwerte, beispielsweise 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74 75 und 76. Gleiches gilt analog für die bevorzugten Temperaturbereiche von 0 - 60 sowie 20-35 °C.

Die Konzentration des Lösungsvermittlers in der zu untersuchenden Probelösung beträgt vorzugsweise 1 - 80 Vol.-% und insbesondere bevorzugt ca. 20 Vol.-%.

Auch bei diesen Bereichsangaben sind alle in den Bereich fallenden Werte und insbesondere ganzzahligen Werte umfasst und offenbart. So umfasst und offenbart der Bereich 1 - 80 Vol.-% zumindest folgende ganzzahligen Einzelwerte: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 und 80.

Die Konzentration an Glycerin in der zu untersuchenden Probelösung kann 1 - 90 m/V, vorzugsweise 5 - 50 m/V und weiterhin bevorzugt ca. 40 m/V betragen. Auch in diesem Fall umfassen die angegebenen Bereiche alle in die angegebenen Bereich fallenden Werte und insbesondere ganzzahligen Werte. So umfasst und offenbart der Bereich für die Konzentration an Glycerin von 5 - 50 zumindest folgende ganzzahligen Werte: 5, 6, 7, 8, 9, 1, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 und 50.

Die Reaktionsdauer der Derivatisierungsreaktion brw. -reaktion kann 10 Minuten - 10 Tage betragen und beträgt vorzugsweise 10 - 20 Stunden, beispielsweise 10, 11, 12, 13, 14, 15, 16, 17, 18 und 20 Stunden, insbesondere bevorzugt 15 Stunden.

Die Konzentration des Derivatisierungsreagenzes, z. B. PFBHA, in der Probelösung beträgt vorzugsweise 0,01 mg/ml bis 100 mg/ml , insbesondere bevorzugt 0,2 mg/ml. Auch in diesem Fall sind alle in den Bereichen liegenden Werte umfasst und offenbart, z. B. 0,1, 0,2, 0,3, 0,4, 0,5, 0,6, 0,7, 0,8, 0,9, 1, 2, 3 usw. bis 100.

Die Umsetzung/Derivatisierung erfolgt vorzugsweise bei einem pH-Wert von 2 bis 7, beispielsweise2, 3, 4, 5, 6 oder 7

Bei der chromatographische Auftrennung (HPLC-Verfahren) wird eine mobile Phase eingesetzt, die aus zwei oder mehr Flüssigkeiten aufweist, insbesondere um eine mobile Phase A, bei der es sich um Wasser oder um eine gepufferte wässrige Phase (bevorzugt) handelt, und um eine mobile Phase B, bei der es sich um organisches Lösungsmittel oder Lösungsmittelgemisch handelt.

Als Puffer können solche eingesetzt werden, die auf dem chromatographischen Gebiet bekannt und üblicherweise eingesetzt werden.

Als organisches Lösungsmittel kann ein polares protisches Lösungsmittel wie Essigsäure, Methanol, Ethanol, n-Propanol oder Isopropanol oder ein polares aprotisches Lösungsmittel wie Aceton, Acetonitril, Dimethoxyethan, DMF, DMSO, 1,4-Dioxan, Pyridin oder THF eingesetzt werden. Vorzugsweise wird Acetonitril verwendet.

Der Gradient bei der chromatographischen Auftrennung wird derart gewählt, dass die relativen Konzentrationen der mobilen Phase bzw. Flüssigkeiten A und B in den ersten 1 bis 100 Minuten zwischen 100 %A : 0%B und 60 % A:40 % B liegen und sich innerhalb von 0 bis 200 Minuten derart ändern, dass sie zwischen 60% A : 40% B und 0%A :100% B liegen.

Nach der chromatographischen Auftrennung erfolgt eine Detektion der erhaltenen Reaktionsprodukte, wodurch eine mengenmäßige Bestimmung vorgenommen wird. Bei dieser Detektion handelt es sich vorzugsweise um eine UV-Detektion.

Die UV-Detektion wird vorzugsweise bei einer Wellenlänge von 180-400 nm, weiterhin bevorzugt bei 190-250 nm und am meisten bevorzugt bei ca. 200 nm vorgenommen. Auch diesen Bereich offenbaren bzw. umfassen alle in die Bereiche fallenden, zumindest ganzzahligen Werte. So umfasst und offenbart der Bereich 190-250 nm zumindest folgenden ganzzahligen Werte: 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 und 250.

Durch die gewählte chromatographische Aufarbeitung und Detektion ist es möglich, den Überschuss an Derivatisierungsreagenz von den Keton- und Aldehydderivaten zu trennen. Ferner können die Keton- und Aldehydderivate untereinander aufgetrennt werden. Dadurch wird eine selektive Bestimmung jedes bekannten Derivats (beispielsweise Glycerinaldehyd (GA), Dihydroxyaceton (DHA), Hydroxyaceton (HA) und Formaldehyd (FA)) und sonstiger möglicher Derivate aus Oxydationsprodukten im Glycerin ermöglicht.

Das erfindungsgemäße Verfahren dient insbesondere zur quantitativen Bestimmung von Glycerinaldehyd, Dihydroxyaceton, Hydroxyaceton und Formaldehyd sowie sonstiger möglicher Oxidationsprodukte in Form von Aldehyden und Ketonen in Glycerin. Diese Aldehyde und Ketone können in dem untersuchten Glycerin natürlich nebeneinander vorliegen.

Die Gehalte der verschiedenen Verunreinigungen werden vorzugsweise durch Vergleich mit einer dieser Verunreinigungen enthaltenden, derivatisierten Kalibrierlösung bzw. Kalibrierprobe errechnet. Mit anderen Worten, eine bekannte Lösung mit genau festgelegten Gehalten an zu bestimmenden Verunreinigungen wird ebenso behandelt und derivatisiert wie eine zu untersuchende flüssige Probe bzw. Probelösung.

Mit Hilfe des erfindungsgemäßen Verfahrens ist es möglich, bei gegebener Matrix Glycerin eine vollständige Umsetzung der Verunreinigungen in der Matrix zu erzielen, ohne dass irgendwelche Komponenten ausfallen. Zudem ist es möglich, eine vollständige Umsetzung der Verunreinigungen zu erreichen, ohne Zersetzungsreaktionen hervorzurufen.

Gegenstand der Erfindung ist auch die Verwendung von Glycerin, bei dem mittels des in der vorliegenden Anmeldung beschriebenen Verfahrens ein Gehalt an Verunreinigungen von 9 ppm oder weniger, beispielsweise 9, 8, 7, 6, 5, 4, 3, 2 und1, bestimmt wurde, zur Herstellung eines Arzneimittels, insbesondere eines in der EP 1242121 B1 beschriebenen Polypeptids, insbesondere Insulin.

Das erfindungsgemäße Verfahren wird nachstehend anhand eines konkreten Beispiels näher erläutert.

Für die Durchführung des Verfahrens werden folgende Reagenzien hergestellt:
Mobile Phasen:
   A: 100 µl Phosphorsäure 85 % in 11 Wasser (1I für ca. 40 Läufe)
   B: Acetonitril
Derivatisierungslösung:
   0,25 g PFBHA (O-(2,3,4,5,6-pentafluorobenzyl)hydroxylamin hydrochlorid werden in einem Messkolben in 25,0 ml Pufferlösung pH = 4
   (z.B. Certipur Citrate/NaOH/HCI, Fa. Merck) gelöst.
Stammlösung 1 (1000 ppm):
   In einen 100 ml Messkolben werden ungefähr 95 ml Wasser gegeben. Je ca. 100 mg Glycerinaldehyd und Dihydroxyaceton sowie je 100 µl Hydroxyaceton und Formaldehydlösung (35%) werden zugewogen. Der Kolben wird mit Wasser zu 100,0 ml aufgefüllt. Die Feststoffe werden vollständig gelöst, hierfür kann ein Ultraschall verwendet werden.
Stammlösung 2 (10 ppm):
   1,0 ml Stammlösung 1 wird in einen 100 ml Messkolben pipettiert und mit Wasser zu 100,0 ml aufgefüllt.
Kalibrationsstandard Glyc-PFBHA-0.40:
   1,0 ml Stammlösung 2, 5,0 ml Acetonitril und 0,5 ml Derivatisierungslösung werden in einem 25 ml Messkolben mit Wasser zu 25,0 ml aufgefüllt.
Kalibrationsstandard Glyc-PFBHA-4.00:
   10,0 ml Stammlösung 2, 5,0 ml Acetonitril und 0,5 ml Derivatisierungslösung werden in einem 25 ml Messkolben mit Wasser zu 25,0 ml aufgefüllt.
Blindlösung:
   5,0 ml Acetonitril und 0,5 ml Derivatisierungslösung werden in einem 25 ml Messkolben mit Wasser zu 25,0 ml aufgefüllt.
Probelösung:
   10 g Probe, 5,0 ml Acetonitril und 0,5 ml Derivatisierungslösung werden in einem 25 ml Messkolben mit Wasser zu 25,0 ml aufgefüllt.
Aufgestockte Probe:
   10 g Probe (Glycerin), 5,0 ml Stammlösung 2, 5,0 ml Acetonitril und 0,5 ml Derivatisierungslösung werden in einem 25 ml Messkolben mit Wasser zu 25,0 ml aufgefüllt.

Die Derivatisierung der verschiedenen Lösungen erfolgt in einem Autosampler einer HPLC-Anlage bei 25°C. Die Derivatisierung ist vollständig, wenn die relative Standardabweichung dreier aufeinander folgender Messungen des Kalibrationsstandards Glyc-PFBHA-4,00 nicht mehr als 2 % beträgt.

Die analytische Bestimmung erfolgt flüssigchromatographisch unter Vorgabe der folgenden HPLC-Bedingungen:
- Säule:: Discovery C18, 25 cm x 4 mm, 5µm
- Injektionsvolumen:: 20 µL
- Analysedauer:: 85 min
- Säulentemperatur:: 25°C
- Autosamplertemperatur:: 25°C
- Detektor:: Diodenarraydetektor
- Wellenlänge:: 200nm
Gradient: lineare Rampen

| Zeit (min) | | % B |
|---|---|---|
| 0 | 20 | |
| 2 | 20 | |
| 60 | 100 | |
| 75 | 100 | |
| 78 | 20 | |

| Zeit (min) | Fluss (ml/min) |
|---|---|
| 0 | 0,5 |
| 60 | 0,5 |
| 65 | 1,0 |
| 79 | 1,0 |
| 80 | 0,5 |

Die Reihenfolge der Injektionen ist folgende:

| Lösung | Anzahl der Injektionen |
|---|---|
| Glyc-PFBHA-4.0 | bis relative Standardabweichung von GA, DHA und HA von 3 aufeinanderfolgenden Messungen kleiner als 2 % ist (ca. 10 Messungen) |
| Blindlösung | 1 Messung |
| Glyc-PFBHA-0.4 (Kalibrierung) | 1 Messung |
| Glyc-PFBHA-4.0 (Kalibrierung) | 1 Messung |
| Aufgestockte Probe | 1 Messung |
| Probe | 1 Messung |
| Weitere Proben | x Messungen |
| Glyc-PFBHA-4.0 (Drift check) | 1 Messung |
| | |

Auf Basis obiger Daten werden Kalibrationsgeraden berechnet. Die Gehalte an Glycerinaldehyd, Dihydroxyaceton, Hydroxyaceton und Formaldehyd sowie sonstiger möglicher Oxidationsprodukte in der Probe beziehungsweise in den Proben werden anhand der ermittelten Kalibrationsgeraden berechnet.

Diese Methode ist gemäß ICH¹ Q2 (R1) (VALIDATION OF ANALYTICAL PROCEDURES: TEXT AND METHODOLOGY) validiert.

Mit Hilfe des erfindungsgemäßen Verfahrens werden die im Glycerin enthaltenden Verunreinigungen und Abbauprodukte wesentlich präziser erfasst als gemäß den bisher bekannten, in Arzneibuchmonographien beschriebenen Methoden. Dies erhöht die Sicherheit bei der Herstellung sensibler Arzneimittel.

## Patentansprüche

1. Verfahren zur quantitativen Bestimmung von Verunreinigungen in Form von Aldehyden und Ketonen in für die Arzneimittelherstellung dienendem Glycerin, bei dem das die Verunreinigungen enthaltende Glycerin in einer Probelösung mit einem Derivatisierungsreagenz umgesetzt und die Menge an derivatisierten Verunreinigungen bestimmt werden,
**dadurch gekennzeichnet, dass**
als Derivatisierungsreagenz PFBHA, O-(2,3,4,5,6-pentafluorobenzyl)-hydroxylamin hydrochlorid, eingesetzt wird,
die Derivatisierung in Anwesenheit eines Lösungsvermittlers in Form eines polaren organischen Lösungsmittels durchgeführt wird und eine flüssigchromatographische Auftrennung sowie eine Detektion der aufgetrennten Verunreinigungen vorgenommen werden.

2. Verfahren nach Anspruch 1,
**dadurch gekennzeichnet, dass**
es sich bei den Verunreinigungen um Glycerinaldehyd, Dihydroxyaceton, Hydroxyaceton und Formaldehyd handelt.

3. Verfahren nach Anspruch 1 oder 2,
**dadurch gekennzeichnet, dass**
Acetonitril als Lösungsvermittler eingesetzt wird und/oder nach der flüssigchromatographische Auftrennung eine UV-Detektion vorgenommen wird.

4. Verfahren nach einem der vorhergehenden Ansprüche,
**dadurch gekennzeichnet, dass**
die UV-Detektion bei einer Wellenlänge von 180 bis 400 nm vorgenommen wird.

5. Verfahren nach Anspruch 4,
**dadurch gekennzeichnet, dass**
die UV-Detektion bei einer Wellenlänge von 190 bis 250 nm, insbesondere bei ca. 200 nm vorgenommen wird.

6. Verfahren nach einem der vorhergehenden Ansprüche,
**dadurch gekennzeichnet, dass**
die Derivatisierung in einem thermostatisierten Raum durchgeführt wird.

7. Verfahren nach einem der vorhergehenden Ansprüche,
**dadurch gekennzeichnet, dass**
die Derivatisierung bei 0 bis 76°C durchgeführt wird.

8. Verfahren nach Anspruch 7,
**dadurch gekennzeichnet, dass**
die Derivatisierung bei ca. 25°C durchgeführt wird.

9. Verfahren nach einem der vorhergehenden Ansprüche,
**dadurch gekennzeichnet, dass**
die Konzentration des Lösungsvermittlers in der Probelösung 1 bis 80 Vol.-% beträgt.

10. Verfahren nach Anspruch 9,
**dadurch gekennzeichnet, dass**
die Konzentration des Lösungsvermittlers in der Probelösung ca. 20 Vol.-% beträgt.

11. Verfahren nach einem der vorhergehenden Ansprüche,
**dadurch gekennzeichnet, dass**
die Konzentration an Glycerin in der Probelösung 5 bis 50 % (m/V) und insbesondere ca. 40 % (m/V) beträgt.

12. Verfahren nach einem der vorhergehenden Ansprüche,
**dadurch gekennzeichnet, dass**
die Reaktionsdauer der Derivatisierungsreaktion 10 Minuten bis 20 Stunden beträgt.

13. Verfahren nach einem der vorhergehenden Ansprüche,
**dadurch gekennzeichnet, dass**
bei der flüssigchromatographische Auftrennung zwei mobile Phasen A und B eingesetzt werden,
als mobile Phase A Wasser oder eine gepufferte wässrige Lösung und als mobile Phase B ein organisches Lösungsmittel oder Lösungsmittelgemisch und, insbesondere Acetonitril eingesetzt werden.

14. Verfahren nach einem der vorhergehenden Ansprüche,
**dadurch gekennzeichnet, dass**
die Gehalte an Verunreinigungen durch Vergleich mit einer diese Verunreinigungen enthaltenden, derivatisierten Kalibrierlösung errechnet werden.

## Claims

1. A method for quantitatively determining impurities in the form of aldehydes and ketones in glycerin serving for preparing pharmaceuticals, in which the glycerin containing impurities is reacted with a derivatization reagent in a sample solution and the quantity of derivatized impurities is determined, **characterized in that**
PFBHA, O-(2,3,4,5,6-pentafluorobenzyl)-hydroxylamine hydrochloride,
is used as the derivatization reagent,
the derivatization is conducted in the presence of a solubilizer in the form of a polar organic solvent, and
a liquid chromatographic separation and a detection of the separated impurities are performed.

2. The method according to claim 1,
**characterized in that**
the impurities are glyceraldehyde, dihydroxyacetone, hydroxyacetone and formaldehyde.

3. The method according to claim 1 or 2,
**characterized in that**
acetonitrile is used as the solubilizer and/or UV detection is performed after the liquid chromatographic separation.

4. The method according to any one of the preceding claims,
**characterized in that**
the UV detection is conducted at a wavelength of 180 to 400 nm.

5. The method according to claim 4,
**characterized in that**
the UV detection is conducted at a wavelength of 190 to 250 nm. In particular at about 200 nm.

6. The method according to any one of the preceding claims,
**characterized in that**
the derivatization is performed in a thermostated space.

7. The method according to any one of the preceding claims,
**characterized in that**
the derivatization is performed at 0 to 76°C.

8. The method according to claim 7
**characterized in that**
the derivatization is performed at about 25°C.

9. The method according to any one of the preceding claims,
**characterized in that**
the concentration of the solubilizer in the sample solution is 1 to 80 vol%.

10. The method according to claim 9,
**characterized in that**
the concentration of the solubilizer in the sample solution is about 20 vol%.

11. The method according to any one of the preceding claims,
**characterized in that**
the concentration of glycerin in the sample solution is 5 to 50% (m/v), and in particular about 40% (m/v).

12. The method according to any one of the preceding claims,
**characterized in that**
the reaction time of the derivatization reaction is 10 minutes to 20 hours.

13. The method according to any one of the preceding claims,
**characterized in that**
two mobile phases A and B are used in the liquid chromatographic separation,
water or a buffered aqueous solution is used as the mobile phase A, and an organic solvent or solvent mixture and in particular acetonitrile is used as the mobile phase B.

14. The method according to any one of the preceding claims,
**characterized in that**
the content of impurities is calculated by comparing to a derivatized calibrating solution containing said impurities.

## Revendications

1. Procédé de détermination quantitative d'impuretés sous forme d'aldéhydes et de cétones dans la glycérine servant à la fabrication de médicaments, dans lequel la glycérine contenant les impuretés est mise en réaction dans une solution d'essai avec un réactif de dérivatisation et la quantité d'impuretés dérivatisées est déterminée, **caractérisé en ce que**,
comme réactif de dérivatisation, on utilise PFBHA, le chlorhydrate de O-(2,3,4,5,6-pentofluorobenzyl)-hydroxylamine,
la dérivatisation est effectuée en présence d'un agent de solubilisation sous forme d'un solvant organique polaire et
une séparation par chromatographie en phase liquide ainsi qu'une détection des impuretés séparées sont effectuées.

2. Procédé selon la revendication 1,
**caractérisé en ce que**,
en matière d'impuretés, il s'agit de glycéraldéhyde, dihydroxyacétone, hydroxyacétone et formaldéhyde.

3. Procédé selon la revendication 1 ou 2,
**caractérisé en ce que**
de l'acétonitrile est utilisé comme agent de solubilisation et/ou **en ce qu'**une détection UV est effectuée après la séparation par chromatographie en phase liquide.

4. Procédé selon l'une des revendications précédentes,
**caractérisé en ce que**
la détection UV est effectuée à une longueur d'onde de 180 à 400 nm.

5. Procédé selon la revendication 4,
**caractérisé en ce que**
la détection UV est effectuée à une longueur d'onde de 190 à 250 nm, en particulier d'environ 200 nm.

6. Procédé selon l'une des revendications précédentes,
**caractérisé en ce que**
la dérivatisation est effectuée dans un espace thermostaté.

7. Procédé selon l'une des revendications précédentes,
**caractérisé en ce que**
la dérivatisation est effectuée entre 0 et 76° C.

8. Procédé selon la revendication 7,
**caractérisé en ce que**
la dérivatisation est effectuée à environ 25° C.

9. Procédé selon l'une des revendications précédentes,
**caractérisé en ce que**
la concentration de l'agent de solubilisation dans la solution d'essai est de 1 à 80 % en volume.

10. Procédé selon la revendication 9,
**caractérisé en ce que**
la concentration de l'agent de solubilisation dans la solution d'essai est d'environ 20 % en volume.

11. Procédé selon l'une des revendications précédentes,
**caractérisé en ce que**
la concentration en glycérine dans la solution d'essai est de 5 à 50 % (m/V) et en particulier d'environ 40 % (m/V).

12. Procédé selon l'une des revendications précédentes,
**caractérisé en ce que**
la durée de réaction de la réaction de dérivatisation est de 10 minutes à 20 heures.

13. Procédé selon l'une des revendications précédentes,
**caractérisé en ce que**,
lors de la séparation par chromatographie en phase liquide, deux phases mobiles A et B sont utilisées,
de l'eau ou une solution aqueuse tamponnée comme phase mobile A,
et un solvant ou mélange de solvant organique et en particulier de l'acétonitrile comme phase mobile B.

14. Procédé selon l'une des revendications précédentes,
**caractérisé en ce que**
les teneurs en impuretés sont calculées par comparaison avec une solution d'étalonnage dérivatisée contenant ces impuretés.
